(19) Europäisches Patentamt
European Patent Office
Office européen des brevets

(11) Publication number: **0 101 786**
**B1**

(12) # EUROPEAN PATENT SPECIFICATION

(45) Date of publication of patent specification: **04.02.87**

(51) Int. Cl.$^4$: **A 61 K 31/425,** C 07 D 275/04

(21) Application number: **83103522.5**

(22) Date of filing: **12.04.83**

(54) **Use of a benzoisothiazoline derivative for the manufacture of a medicament against thrombosis and platelet aggregation.**

(30) Priority: **12.04.82 JP 61489/82**

(43) Date of publication of application:
**07.03.84 Bulletin 84/10**

(45) Publication of the grant of the patent:
**04.02.87 Bulletin 87/06**

(84) Designated Contracting States:
**BE DE GB NL SE**

(56) References cited:
**EP-A-0 000 254**
**DE-A-2 753 391**
**GB-A- 848 130**
**GB-A-1 495 909**
**US-A-3 300 378**

**Patent Abstracts of Japan vol. 6, no. 197, 06 October 1982**

(73) Proprietor: **THE GREEN CROSS CORPORATION**
**15-1, Imabashi-1-chome Higashi-ku Osaka-shi**
**Osaka 541 (JP)**

(72) Inventor: **Yokoyama, Kazumasa**
**No. 7, Terauchi 2-chome**
**Toyonaka-shi Osaka (JP)**
Inventor: **Watanabe, Masahiro**
**No. 570-22, Ookuradani Shimizu**
**Akashi-shi Hyogo (JP)**
Inventor: **Takanabe, Atsuyuki**
**No. 20-18, Nagaodai 1-chome**
**Hirakata-shi Osaka (JP)**
Inventor: **Suyama, Tadakazu**
**No. 3-7, Matsuigaoka 4-chome Tanabe-cho**
**Tsuzuki-gun Kyoto (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 101 786 B1**

**Description**

Thrombi are blood clots and their formation can cause thrombosis. It is generally agreed that thrombi are formed primarily due to the coagulation of blood and various activities of platelets such as adhesion, aggregation, and release of adenosine diphosphate (ADP) (see, for example, *Ketsueki to Myakukan* (Blood and Vessels), *1*, 4, pp. 13—24, June 1970). Based on the observation that these functions of platelets participate directly in the formation of thrombi, attempts have already been made to prevent formation of thrombi by inhibiting the activities of platelets. Among the drugs that are currently under review as potential antithrombotic agents are adenosine derivatives, dextran, dipyridamole derivatives and aspirin anti-inflammatories, but as far as is known none of them have been reported to have a significant efficacy.

As a result of various studies to develop an effective anti-thrombotic agent, it has unexpectedly been found that some benzoisothiazoline derivatives are capable of inhibiting the aggregation of platelets, that they are more effective than known antithrombotic agents against platelet aggregation induced by ADP, and that they have higher $LD_{50}$ levels than known antithrombotic agents in acute toxicity tests. The present invention has been accomplished based on the above findings.

The present invention relates to the use of a compound of general formula

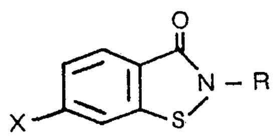

(I)

wherein R represents a hydrogen atom or an alkyl group having not more than 10 carbon atoms; and X is a halogen atom; for the manufacture of a medicament against thrombosis and platelet aggregation.

The alkyl group represented by R can be any of straight chain or branched chain alkyl groups having 1 to 10 carbon atoms. Suitable examples of the alkyl group represented by R include methyl, ethyl, n-propyl, iso-propyl, n-butyl, iso-butyl, tert-butyl, n-pentyl, n-hexyl, n-heptyl, n-octyl, n-nonyl and n-decyl, with n-propyl being particularly preferred. Examples of the halogen atom represented by X include chlorine and bromine.

Among the compounds of general formula (I) (hereinafter referred to as "BIT (1)" for brevity), the one in which R represents a hydrogen atom and X represents a chlorine atom is described in GB—A—848,130 and DE—A—1,135,468 and is known to have a microorganism controlling activity. Compounds encompassed by the general formula (I) are disclosed in US—A—3 517 022 and US—A—3 862 955 as being useful as bactericides and fungicides. Furthermore, it is disclosed in US—A—3 300 378 that 6-chloro-1,2-benzisothiazolone is suitable for use as antiseptic and anti-inflammatory agent.

Illustrative examples of BIT (1) compounds which can be used for the manufacture of a medicament against thrombosis include 6-chloro-2-n-propyl-1,2-benzoisothiazolin-3-one and 6-chloro-2-n-decyl-1,2-benzoisothiazolin-3-one.

The BIT (1) of the present invention can be prepared by one of the following two methods.

(Method 1)

The first method comprises reacting a compound of formula (II):

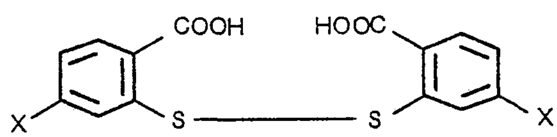

(II)

wherein X is the same as defined in general formula (I) or a reactive derivative thereof (e.g. acid halide such as acid chloride) with a compound of general formula (III):

$$R—NH_2 \qquad (III)$$

wherein R is the same as defined in general formula (I) to form a compound of general formula (IV):

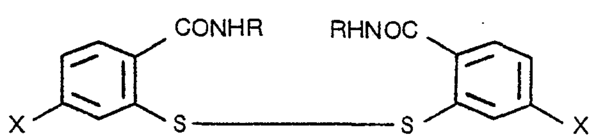

(IV)

wherein X and R are the same as defined in formula (I), and cyclizing this compound (IV) after optionally isolating it from the reaction mixture.

In Method 1, the reaction between the compound of general formula (II) and the compound of the general formula (III) is effected in the presence of an inert organic solvent such as benzene, carbon tetrachloride or dioxane. The reaction temperature generally ranges from 20 to 100°C, preferably from 30 to 70°C, and more preferably from 40 to 60°C. Compound of general formula (IV) may be cyclized by first adding a halogen atom (e.g. chlorine or bromine) to said compound and heating the addition product. The addition of a halogen atom can be performed by a known method which comprises adding a halogen solution (preferably a bromine solution) to compound (IV) in the presence of an organic solvent such as carbon tetrachloride. This addition reaction can be carried out at room temperature (10 to 30°C) under stirring. The heat treatment following the addition of halogen atom is effected at an elevated temperature between 70° and 150°C, for example heating can usually be performed under refluxing in the presence of an organic solvent such as acetic acid. The refluxing period of about 30 minutes will serve the purpose.

(Method 2)

The second method comprises reacting a compound of general formula (V):

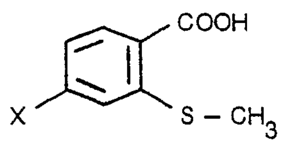

(V)

wherein X is the same as defined in formula (I) or a reactive derivative thereof (the same as defined for compound (II)) with a compound of general formula (III) to form a compound of general formula (VI):

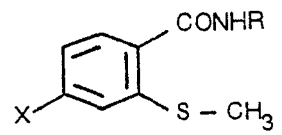

(VI)

wherein X and R are each the same as defined in formula (I), oxidizing a compound of general formula (VI) after optional isolation to obtain a compound of general formula (VII):

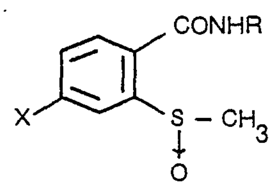

(VII)

wherein X and R are each the same as defined in formula (I), and cyclizing this compound after optional isolation from the reaction mixture.

In Method 2, the reaction between the compound of general formula (V) and the compound of general formula (III) is effected in the presence of an inert organic solvent such as benzene, carbon tetrachloride, dioxane or chloroform. The reaction temperature generally ranges from 20 to 100°C, preferably from 30 to 70°C, and more preferably from 40 to 60°C. Compound of general formula (VI) can be oxidized with a suitable oxidizing agent such as periodic acid, hypohalogenous acid ester or hydrogen peroxide. Compound of general formula (VII) can be cyclized by heating at a temperature usually ranging between 50 and 100°C. The heating can generally be effected in a water bath in the presence of 2N-NaOH.

The BIT (1) thus obtained can be isolated and purified by a known method such as recrystallization from ethanol or petroleum ether or silica gel chromatography using an eluting solution composed of benzene, dioxane and acetic acid (90:20:2).

*Preparation of 6-chloro-2-n-propyl-1,2-benzoisothiazoline-3-one*

To a suspension of 37.5 g (0.1 mol) of bis(5-chloro-2-carboxyphenyl)disulfide in benzene (100 ml), 35.7 g (0.3 mol) of thionyl chloride was added, and the mixture was heated for 12 hours under reflux. The benzene and excess thionyl chloride were removed under vacuum, and the residue was dissolved in 600 ml of dioxane. The solution was added dropwise to a solution of 30 g (0.5 mol) of n-propylamine in 50 ml of dioxane at 40 to 60°C with stirring. As the reaction proceeded, the reaction mixture turned into a suspension. The suspension was agitated at 40 to 60°C for 30 minutes and thereafter it was cooled. The cooled suspension was poured into 6 liters of ice water, and mixed with about 60 ml of 2N-HCl to adjust its pH to 3. The resulting crystal was filtered out and dried.

The dried product (4.1 g) was suspended in 400 ml of carbon tetrachloride, and to the suspension, a

solution of 18 g (0.23 mol) of bromine in 50 ml of carbon tetrachloride was added dropwise at room temperature. The mixture was stirred at room temperature for 30 minutes, and the resulting orange yellow crystal was filtered out, and suspended in 400 ml of glacial acetic acid. The suspension is stirred at 120 to 140°C to form a solution. The solution was heated at 120 to 140°C for 30 minutes with stirring and was thereafter cooled. The cooled solution was poured into 4 liters of ice water. The resulting crystals were filtered and washed three times with 200 ml portions of water. Pale brown acicular crystals (32.3 g; yield: 86.1%) of the title compound having m.p. 76 to 78°C was obtained. Two recrystallizations from water-ethanol (1:2) gave colorless acicular crystals having m.p. 78 to 79°C. An elemental analysis of the crystal is given in Table 2 below.

TABLE 2
Elemental analysis for $C_{10}H_{10}NOSCl$

| | C(%) | H(%) | N(%) | S(%) | Cl(%) |
|---|---|---|---|---|---|
| Calculated: | 52.72 | 4.43 | 6.15 | 14.08 | 15.57 |
| Found: | 52.79 | 4.51 | 6.22 | 14.11 | 15.55 |

Experiments to confirm the pharmacological effects, effective dose, route of administration and toxicity of BIT (1) compounds were conducted. The procedures and results of these tests are described below.

(1) *Inhibition of Platelet Aggregation*

(a) Effectiveness against ADP-induced aggregation

The inhibitory activity of BIT (1) compounds against platelet aggregation initiated by promoter ADP was determined for various amounts of the BIT (1) compounds. Platelet aggregation was observed with Aggregometer of Bryston Corp. and the degree of aggregation was measured by nephelometry in terms of the change in absorbance [*Rinsho Kensa* (Journal of Medical Technology), *17*, 11, Supplement, pp. 187—190, November 1963]. Silicon-coated cuvettes on the Aggregometer were filled with 0.8 ml of diluted platelet-rich plasma (containing 300,000 to 400,000 platelets per $mm^3$) and 0.1 ml of solutions of the BIT (1) compounds listed in Table 1 having different concentration (0.3 μg/ml, 1.0 μg/ml and 3.0 μg/ml as dissolved in 100% alcohol), as well as adenosine (20 μg/ml and 100 μg/ml) and aspirin (500 μg/ml) were added. Following 2-minute incubation, ADP was added to give a final concentration of $10^{-5}$ M. The final volume of each sample was 1 ml. The process of platelet aggregation was monitored with the recorder pen which traced aggregation curves.

The percent inhibition of aggregation was calculated by the following formula:

$$\frac{M - D}{M} \times 100(\%)$$

wherein M represents light transmittance for maximum aggregation in 100% ethanol (Control);

D represents light transmittance for maximum aggregation in the specific sample.

The test results are shown in Table 1 below.

TABLE 1

| Antithrombotic agent | Amount (final conc.) | Inhibition of platelet aggregation | Melting point |
|---|---|---|---|
| BIT (R = n-$C_3H_7$) | 0.3 µg/ml | 18% | |
| (X = Cl) | 1.0 µg/ml | 43% | 78—79°C |
| | 3.0 µg/ml | 100% | |
| BIT (R = H) | 10.0 µg/ml | 100% | 273—276°C |
| (X = Cl) | | | |
| BIT (R = n-$C_6H_{13}$) | 3.0 µg/ml | 100% | 86—87°C |
| (X = Cl) | | | |
| BIT (R = n-$C_{10}H_{21}$) | 3.0 µg/ml | 100% | 85—87°C |
| (X = Cl) | | | |
| Adenosine | 2 µg/ml | 47% | |
| | 10 µg/ml | 62% | |
| Aspirin | 50 µg/ml | 0% | |
| 100% Ethanol (Control) | | 0% | |

As the above data shows, the BIT (1) compounds of the present invention are highly effective in suppressing the ADP-induced aggregation of platelets. Having this feature, the BIT (1) compounds will prove useful as an antithrombotic agent to prevent the formation of thrombi that are thought to be closely related to the functions of factors making up human platelets.

(2) *Acceleration of Platelet Disaggregation*

ADP was added to platelet-rich plasma as used in (1) above, and when maximum aggregation was reached, BIT (1) compound (R = n-$C_3H_7$, X = Cl) was added to check its ability to accelerate the disaggregation of platelets. In this experiment, the procedure of experiment (1) was repeated except that the BIT (1) was added after the aggregation of platelets occurred. To give final concentrations of 1.0 µg/ml, 3.0 µg/ml and 6.0 µg/ml as dissolved in 100% ethanol, 0.1 ml of solutions containing 10 µg/ml, 30 µg/ml and 60 µg/ml of BIT (1) were added after maximum platelet aggregation took place. As a control, 100% ethanol was added instead of the BIT (1). The degree of disaggregation was determined by measuring the absorbance of the test samples 5 minutes after the addition of the BIT (1) as against the absorbance for maximum aggregation in that sample. The control had a disaggregation level of about 12%, but the test samples containing BIT (1) achieved higher disaggregation levels: final BIT (1) concentrations of 1.0 µg/ml, 3.0 µg/ml and 6.0 µg/ml gave disaggregation levels of 25%, 47% and 64%.

(3) *Acute Toxicity Test*

A group of 10 male ddy mice each weighing 200 g were subject to an acute toxicity test with BIT (1) (R = n-$C_3H_7$, X = Cl) which was administered to the mice orally as suspensions in distilled water for injection that contained 0.5% of carboxymethyl cellulose. The $LD_{50}$ of the BIT (1) as determined by the Litchfield-wilcoxon method was 6,500 mg/kg, and this demonstrates the very low toxicity of the BIT (1).

(4) *Effective dose and route of administration*

As demonstrated in the acute toxicity test, the BIT (1) of the present invention is a highly safe compound and there is no particular upper limit to the dose of the compound. Generally, it is administered daily in an amount of 10 mg to 40 mg/kg per adult, either in a single dose or several doses.

The antithrombotic agent containing the BIT (1) of the present invention can be administered either as an injection or as an oral preparation. A suitable injection may be prepared from an emulsion in oil, or by dissolving the BIT (1) in a nonionic surfactant such as polyethylene glycol (av. mol. wt. 400—600), Tween® 80 or Pluronic® F 68. Illustrative examples of oral preparations include capsules, tablets, powders,

and oral liquid formulations. Specific injections and oral preparations may be produced by conventional pharmaceutical techniques described, for example, in the Japanese Pharmacopeia.

The BIT (1) incorporated as the effective ingredient in the antithrombotic agent of the present invention has a very low toxicity and yet exhibits significant antithrombotic effects in the human body. The BIT (1) not only inhibits the aggregation of platelets but also accelerates their disaggregation. This suggests that a drug containing BIT (1) is useful for preventing and treating thrombosis. The BIT (1) may be used with a thrombolytic agent and because of their different modes of action, the combination of these two drugs will prove very effective in treating thrombosis.

Two typical examples of the pharmaceutical preparation incorporating the BIT (1) are described hereunder.

*Basic Formulation 1 (Tablets)*

| | |
|---|---|
| BIT (1) | 500 g |
| Lactose | 500 g |
| Potato starch | 315 g |
| Polyvinyl alcohol | 20 g |
| Magnesium stearate | 15 g |
| Total | 1,350 g |

The measured amounts of BIT, lactose and potato starch are mixed uniformly. The resulting mix is further blended with a solution of the polyvinyl alcohol in water (450 ml), and granules are prepared from the blend by the wet granulation method. The granules are mixed with the magnesium stearate and compressed into tablets (3.4 mm thick and weighing 270 mg each) with flat-faced and bevel-edged punches having a diameter of 9 mm.

*Basic Formulation 2 (Capsules)*

| | |
|---|---|
| BIT (1) | 125 g |
| Lactose | 860 g |
| Magnesium stearate | 15 g |
| Total | 1,000 g |

The measured amounts of the above listed ingredients are uniformly mixed. The resulting mix is distributed among 5,000 No. 3 hard gelatin capsules.

*Basic Formulation 3 (5% Powders)*

| | |
|---|---|
| BIT (1) | 50 g |
| Lactose | 950 g |
| Total | 1,000 g |

The measured amounts of the two ingredients are uniformly mixed and powders are prepared from the mix.

The BIT (1) compounds used in the above three formulations can be selected from among those listed in Table 1.

**Claim**

Use of a compound of general formula

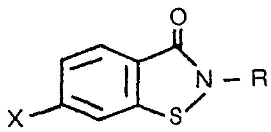

(I)

wherein R represents a hydrogen atom or an alkyl group having not more than 10 carbon atoms; and X is a halogen atom; for the manufacture of a medicament against thrombosis and platelet aggregation.

**Patentanspruch**

Verwendung einer Verbindung der allgemeinen Formel (I)

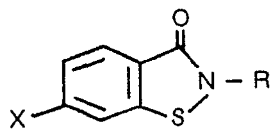

(I)

worin R ein Wasserstoffatom oder eine Alkylgruppe mit nicht mehr als 10 Kohlenstoffatomen bedeutet und X ein Halogenatom ist, zur Herstellung eines Arzneimittels gegen Thrombose und Plättchenaggregation.

**Revendication**

Utilisation d'un composé de formule générale:

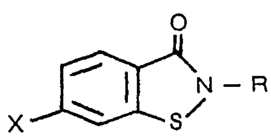

(I)

dans laquelle R représente un atome d'hydrogène ou un groupe alkyle n'ayant pas plus de 10 atomes de carbone, et X représente un atome d'halogène, pour la fabrication d'un médicament contre la thrombose et l'aggrégation de plaquettes.